# EUROPEAN PATENT APPLICATION

(11) **EP 2 267 003 A1**
(43) Date of publication of application: **29.12.2010**
(21) Application number: 09163581.3
(22) Date of filing: 24.06.2009
(51) Int. Cl.: C07K 7/50

(54) **Biologically produced cyclic affinity tags**

(71) Applicant: Applied NanoSystems B.V., 9700 AC Groningen (NL)
(72) Inventor: The designation of the inventor has not yet been filed
(74) Representative: Hatzmann, Martin

(57) **Abstract**

The invention relates to novels ways of introducing an affinity tag into a protein of interest. Provided is an enzymatic method for providing a proteinaceous substance comprising a polypeptide of interest and a cyclic affinity tag, comprising the steps of: (a) providing at least one precursor proteinaceous substance, the precursor comprising said protein of interest and at least one motif of the general formula X1-Tag-X2, wherein X1 and X2 represent amino acids whose side chains can be linked enzymatically by a covalent bond; Tag is an amino acid sequence capable of binding to a binding partner when cyclized; (b) contacting said precursor with at least one enzyme capable of forming a covalent bond between X1 and X2, thereby introducing an intramolecular ring structure comprising the Tag sequence; and (c) isolating the resulting cyclized proteinaceous substance. Also provided are proteinaceous substances obtainable thereby and the use thereof, for instance for preparing a peptide library.

## Description

The invention relates to the field of recombinant protein expression, purification and immobilization. In particular, it relates to novel ways of introducing an affinity tag into a protein of interest.

A wide variety of affinity tags have been developed and are used throughout biotechnology. The widespread success of affinity tags throughout the biological sciences has prompted interest in developing new and convenient labeling strategies. Affinity tags are well-established tools for recombinant protein immobilization and purification. More recently, these tags have been utilized for selective biological targeting towards multiplexed protein detection in numerous imaging applications as well as for drug-delivery. The most commonly employed affinity tags range from short polypeptide sequences, to whole proteins, which can confer advantageous solubility effects. For example, small peptide epitopes such as polyhistidine tags which can bind to immobilized metal chelates, as well as the myc-tag and FLAG-tag, which can bind to immobilized antibodies, are commonly used for the isolation and immobilization of recombinant proteins.

Another small peptide epitope that has gained wide use is the streptavidin specific Strep-tag [Schmidt, et al., Protein Eng. 6 (1993) 109-122; US 2006/0106199 and US 6,841,359.]. Streptavidin binding peptide sequences have been discovered by screening peptide libraries, most but not all of which contain the His-Pro-Gln (HPQ) motif. The development of streptavidin-fusion peptides has aided in a variety of unique biochemical applications and has made streptavidin, the non-glycosylated bacterial relative of avidin, the preferred protein in many applications of the (strept)avidin-biotin technologies [Keefe, et al., Protein Expr. Purif. 23 (2001) 440-446; Lamla, et al., Protein Expr. Purif. 33 (2003) 39-47]. Small peptides such as the Strep-tag can easily be expressed as fusions with larger proteins for use in purification or other conjugation applications. The availability of labeled streptavidin, as well as streptavidin immobilized on solid supports, has made these peptides extremely useful. Using streptavidin as model receptor system, it was found that disulfide-constrained cyclic peptide Strep-motifs bind streptavidin with affinities up to 3 orders of magnitude higher than the corresponding linear sequences (Giebel et al., Biochemistry 1995, 34, 15430-15435). Katz et al.(J. Am. Chem. Soc. 1995, 117, 8541-8547) designed and chemically synthesized cyclic streptavidin binding peptides comprising the motif CHPQGPPC, wherein the disulfide is replaced by a thioether crosslink. The thioether-cross linked peptides were reported to display enhanced stability as compared to their disulfide counterpart.

Thus, cyclic affinity tags like cyclised Strep-motifs have advantageous properties for use as protein tag over their linear counterparts. However, in contrast to fusion proteins comprising linear proteins that can simply be obtained by recombinant protein expression, the provision of a protein comprising a cyclic affinity tag typically requires chemical modification, which is highly undesirable in terms of time, efforts and costs involved. The spontaneous formation of disulfide bridges usually lacks specificity, while the bridges themselves lack stability.

The present inventors therefore set out to provide alternative methods for providing a protein of interest with a cyclised affinity tag. It was found that a linear tag sequence can be cyclised biologically i.e. non-chemically if the tag sequence is flanked on each side by amino acids capable of forming together a covalent bond upon enzyme action, e.g. by an enzyme present in a host cell expressing a construct encoding the protein of interest comprising the tag sequence.

Therefore, the invention relates to a method for providing a proteinaceous substance comprising a protein of interest and a cyclic affinity tag, comprising the steps of:
a) providing at least one precursor proteinaceous substance, the precursor comprising said protein of interest and at least one motif of the general formula X1-Tag-X2 wherein X1 and X2 represent amino acids whose side chains can be linked enzymatically by a covalent bond; Tag is an amino acid sequence capable of binding to a (proteinaceous) binding partner when cyclized; b) contacting said precursor with at least one enzyme capable of forming a covalent bond between X1 and X2, thereby introducing an intramolecular ring structure comprising the Tag sequence; and c) isolating the resulting cyclized proteinaceous substance.

Thus, the invention provides a method involving enzyme-mediated cyclization. As used herein, the expression "enzyme-mediated cyclization" is meant to indicate that at least one step required for ring formation is performed enzymatically i.e. non-chemically. The enzymatic step may be performed *in vivo* or *in vitro.* The enzyme-mediated step(s) may include enzymatic modification of amino acid residues (e.g. dehydration), and/or enzymatic closure of the ring. Enzymatic cyclization may consist of just the enzymatic formation of highly reactive dehydroalanine, but can also consist of cyclase-action (Rink, R., 2007. Biochemistry 46:13179-13189). In one embodiment, steps a) and b) are performed in a host cell comprising said at least one enzyme capable of forming a covalent bond between X1 and X2, said host cell being provided with a nucleic acid sequence encoding said precursor proteinaceous substance. It is also possible to provide the precursor proteinaceous substance by recombinant expression and perform the ring closure by contacting the substance with the appropriate enzyme(s) *in vitro.* Step c) advantageously comprises using an immobilized binding partner, like an antibody or other proteinaceous substance, of the cyclized Tag sequence. For example, affinity chromatography is suitably used.

The polypeptide of interest can be any proteinaceous molecule, including a biologically active polypeptide, such as a hormone, an antimicrobial peptide, receptor agonist, receptor antagonist, or a receptor binding peptide without biological effect. According to the invention, the positioning of the at least one tag sequence (motif) of the general formula X1-Tag-X2 within the proteinaceous substance relative to the polypeptide of interest can vary. In one embodiment, the motif is inserted into the amino acid sequence of the polypeptide of interest ("internal" tag). However, insertion of a foreign sequence can be detrimental for protein function. It may thus be preferred that the motif is added to the polypeptide of interest, e.g. by N- or C-terminal fusion ("external" or "exogenous" tag). In that case, the proteinaceous substance may comprise a cleavage site between said polypeptide of interest and the at least one motif, such that the motif can be removed following step c) by action of the appropriate cleaving enzyme to release the polypeptide of interest. Exemplary cleavage sites included a Factor X or a Glu-C-cleavage site.

In a preferred embodiment, the proteinaceous substance is a polypeptide of interest wherein a portion is replaced by said at least one motif such that the motif is an integral part of said polypeptide of interest ("intrinsic" tag). As will be described below, an intrinsic tag can be realized by replacing a stretch of amino acids being part of a naturally occurring intramolecular ring structure with an amino sequence that encodes (at least when cyclised) an affinity tag while the replacement leaves the desired propertie(s) of the polypeptide intact. This approach is especially suitable for introducing an affinity tag into a biologically produced thioether-bridged peptide, such as nisin or any other type of lantibiotic. An advantage of an intrinsic affinity tag is that it eliminates the need of costly removal of the tag.

Preferably, the Tag sequence consists of 2-20 amino acid residues, more preferably 2-15. Thus, X1 and X2 may be separated by at most 20, preferably at most 15, more preferably 2-8 amino acid residues. Although the invention is exemplified with a cyclized Strep-tag, a method of the invention is not restricted to any type of affinity tag. As used herein, the term affinity tag refers to any sequence capable of binding specifically to a tag-specific binding partner. The binding is typically characterized by a high Kₒₙ and a low K_{off}.

In one embodiment, the Tag sequence comprises or consists of the sequence Arg-Gly-Asp (RGD) and is capable of binding to the glycoprotein IIb/IIIa adhesion molecule. In another embodiment, the Tag sequence is a streptavidin binding sequence having a binding affinity for streptavidin of at least submicromolar Kd. For instance, the streptavidin binding sequence is selected from the group consisting of His-Pro-Gly (HPG), His-Pro-Lys (HPK), His-Pro-Met (HPM), His-Pro-Gln (HPQ), His-Pro-Asn (HPQ) and His-Pro-Gln-Phe (HPQF). In a specific aspect, the streptavidin binding sequence is His-Pro-Gln (HPQ) or His-Pro-Gln-Phe (HPQF). Other useful Tag sequences include DVEAW, DVEAWL/I, DVEA, VEAW, DVE, VEA, EAW, VPLVET, DVXAW, EPDWF/Y, GDF/WXF, PWXWL, VPEY, wherein X is an arbitrary amino acid (see US 6,841,359 and US2008/0032340). Either one of these Tags are advantageously used in a method wherein step c) comprises streptavidin-based affinity chromatography.

The choice of amino acid residues X1 and X2, the residues to form the intracellular ring structure comprising the Tag sequence, will of course depend on the enzyme activitie(s) to be used. For example, if a lantibiotic-enzyme activity is used for enzymatic ring closure, then X1 and X2 can represent residues whose side chains can be converted to a (methyl)lanthionine bridge. In addition, to allow for recognition of the motif by a lantibiotic enzyme, the motif is preceded by a lantibiotic leader sequence. Accordingly, the invention provides an enzymatic method for providing a proteinaceous substance comprising a protein of interest and a cyclic affinity tag, comprising the steps of:
a) providing at least one precursor proteinaceous substance, the precursor comprising said protein of interest and at least one motif of the general formula X1-Tag-X2 wherein X1 is selected from Dhb (dehydrobutyrine), Dha (dehydroalanine), Thr, and Ser and wherein X2 is Cys or Lys; or wherein X1 is Cys or Lys and X2 is selected from Dhb, Dha, Thr and Ser; and wherein said motif is preceded N-terminally by a lantibiotic leader sequence. The distance between the leader sequence and the Tag sequence can vary but is typcially between 0 and 100 amino acids, preferably between 0 and 50 amino acids, more preferentially between 0 and 20 amino acids.
   Tag is an amino acid sequence capable of binding to a binding partner when cyclized;
b) contacting said precursor with at least one lantibiotic enzyme capable of forming a covalent bond between X1 and X2, thereby introducing an intramolecular ring structure comprising the Tag sequence; and
c) isolating the resulting proteinaceous substance comprising a lantibiotic-enzyme-cyclised thioether-bridged affinity tag; the thioether bond being between a D- and an L- amino acid or between an L- and a D-amino acid.

In a preferred embodiment, X1 is Dha or Dhb and X2 is Cys. Suitable lantibiotic leader sequences are well known in the art and include naturally occurring or genetically engineered lantibiotic leader sequences. For instance nisin or lacticin3147 leader sequences. Preferred leader sequences for use in the present invention include leader sequences with a conserved FNLD box at positions -18 to -15 counted from the cleavage site and ending with a factor X -or GluC cleavage site inside the leader. Alternatively, the leader sequence can have conserved boxes ELD at positions -8, -to - 6 and EEV at positions -14 to -12 also having a factor X or GluC cleavage site at its C-terminus. Some aligned leader sequences with conserved boxes for use in the present invention are described in US2009042246.

Various possible lantibiotic enzymes may be used to catalyze ring closure. Partially depending on the nature of X1 and X2, the precursor proteinaceous substance may be contacted with lantibiotic enzyme LanM, cyclase LanC (in the case of a combination of a dehydroresidue and a cysteine) or (e.g. in the case wherein X1 or X2 is Ser or Thr) a combination of a lantibiotic dehydratase LanB and cyclase LanC. A method of the invention is efficiently carried out in a host cell comprising one or more lanthionine-generating enzymes. For example, the host cell comprises the proteins LanB; LanC and LanT; LanM and LanT; LanB and LanC; or only LanM. Suitable host cells are Gram-positive bacteria e.g. *Lactococcus lactis, Bacillus cereus, Streptococcus epidermis, Streptomyces lividans or actinomycetes, e.g. Actinoplanes garbadinensis.* Preferably, the lantibiotic-producing host transformed with the polynucleic acid encoding the precursor proteinaceous substance is a lacticin3147-producing host or a nisin-producing host. More preferably, the lacticin3147-producing host and the nisin-producing host is a strain of *Lactobacillus lactis,* NZ9000. When using a host cell with a lantibiotic enzyme system in which the transporter also has the leaderpeptidase activity, it will be relevant not to comprise the recognition of the novel lantibiotic by the self protection system. In other systems the leaderpeptidase may be left out of the host cell, thus producing the novel lantibiotic with the leaderpeptide still attached to it ensuring inactivity of the prelantibiotic and its harmlessness to the producer cell until removal of the leaderpeptide from the harvested prelantibiotic. In vitro synthesis of thioether rings comprising a streptavidin binding motif by LanM is also possible.

(Methyl)lanthionine-cyclized tag-motif containing polypeptides can be conveniently produced by cells containing a two-plasmid expression system. *lanBTC* or *lanMT* could be encoded by one plasmid, for instance a bidirectionally replicating pIL plasmid. The precursor proteinaceous substance can be encoded by a second plasmid, for instance a pNZ4048 rolling circle replicating plasmid. Having the modification genes and the polynucleotide encoding the protein to be cyclized on the same plasmid is also possible but less practical. Accordingly, in a specific aspect the host cell comprises a first vector encoding said precursor proteinaceous substance and a second vector encoding one or more lantibiotic enzyme(s), for example LanBTC or LanMT. The invention thus also relates to a nucleic acid sequence encoding the precursor proteinaceous substance, a vector comprising said nucleic acid sequence, and a host cell provided with the nucleic acid sequence, preferably being part of a suitable expression vector.

A method of the invention for providing a protein of interest with a thioether-cyclized affinity tag is especially advantageous if the protein of interest itself also (i.e. in addition to the cyclized affinity tag) comprises at least one thioether-containing intramolecular ring structure. For example, it provides an efficient, one-step, and specific procedure for the purification of a biologically produced thioether-bridged peptide. Another attractive application resides in the purification of lantibiotics, wherein the cyclized tag is present as intrinsic tag of the lantibiotic. For instance, it provides nisin with a thioether bridged Strep-tag replacing rings DE. The resulting nisin mutant is readily purified with streptag column. Since nisin A is world wide applied, a drastically improved production method is of great commercial importance. Advantages over existing (i.e. external) tags used for lantibiotic purification are the high affinity allowing high yield, high purity and making the need for costly removal of external tags obsolete.

Yet another application relates to the generation of thioether-peptide libraries immobilized on chips. Thioether bridged peptides may have strongly enhanced therapeutic potential (Kluskens 2009). To obtain stable thioether-bridged peptides, a screening process is generally performed to select for functional peptides. Specific thioether-bridged peptides can be produced in a host cell, like *Lactococcus lactis,* comprising lantibiotic enzymes. However, in the latter case the peptides are not physically linked to their DNA, thus posing a hurdle to the identification of the sequence of the selected peptides. By virtue of a method disclosed in the present invention, thioether-bridged peptides comprising a lantibiotic-enzyme-introduced thioether affinity tag can be biologically produced. Hence, peptides can be produced, identified and immobilized e.g. on a defined spot on multiple identical chips provided with a suitable binding partner of the cyclised tag. This allows for the generation of a library with lantibiotic-enzyme cyclised thioether peptides, which can be screened for specific properties, such as binding or kinase-mediated phosphorylation. In a specific aspect, the polypeptide of interest is a member of a library of thioether-bridged peptides, for example a member of a hexa-, hepta- or octapeptide library. The peptides within library may be screened for a wide variety of properties, including being a kinase substrate or a receptor ligand, such as a Her2-receptor binding peptide or a G3P-receptor binding peptide.

In a preferred embodiment, the motif X1-Tag-X2 is designed to allow for the biological production of a thioether-cyclized streptavidin binding sequence. To that end, the motif may contain a sequence selected from His-Pro-Gly (HPG), His-Pro-Lys (HPK), His-Pro-Met (HPM), His-Pro-Gln (HPQ) and His-Pro-Gln-Phe (HPQF), which sequence is flanked by X1 and X2 wherein X1 is selected from Dhb, Dha, Thr, and Ser and wherein X2 is Cys or Lys; or wherein X1 is Cys or Lys and X2 is selected from Dhb, Dha, Thr and Ser. Preferably, the motif comprises the streptavidin binding sequence His-Pro-Gln (HPQ) or His-Pro-Gln-Phe (HPQF). The inventors found that lantibiotic cyclases can catalyze ring closure of Strep-containing sequences (e.g. DhbHPQFC and DhbHPQFGC). This was unexpected for at least the following reasons: (i) these sequences do not at all occur in natural lantibiotics; (ii) hardly any mutations in nisin's ringC have been published and simultaneous replacement of 4 residues in one lantibiotic thioether ring has not been reported, (iii) the presence of the helix- breaking residue Pro was expected to reduce the likelihood of ring formation.

The X1-Tag-X2 motif consists for example of an amino acid sequence selected from the group consisting of Dha-His-Pro-Gln-Phe-Cys; Dhb-His-Pro-Gln-Phe-Cys; Ser-His-Pro-Gln-Phe-Cys; Thr- His-Pro-Gln-Phe-Cys; Cys-His-Pro-Gln-Phe-Dha; Cys-His-Pro-Gln-Phe-Dhb; Cys-His-Pro-Gln-Phe-Ser; Cys- His-Pro-Gln-Phe-Thr; Dha- His-Pro-Gln-Cys; Dhb-His-Pro-Gln-Cys; Ser-His-Pro-Gln-Cys; Thr-His-Pro-Gln-Cys; Cys-His-Pro-Gln-Dha; Cys-His-Pro-Gln-Dhb; Cys- His-Pro-Gln-Ser; Cys-His-Pro-Gln-Thr; Ser-His-Pro-Gln-Phe -Lys; Thr- His-Pro-Gln-Phe-Lys; Lys-His-Pro-Gln-Phe -Ser; Lys-His-Pro-Gln-Phe-Thr; Dha- His-Pro-Gln-Phe-Lys; Dhb-His-Pro-Gln-Phe-Lys; Lys-His-Pro-Gln -Dha; Lys- His-Pro-Gln-Dhb; Ser- His-Pro-Gln-Lys; Thr-His-Pro-Gln-Lys; Lys-His-Pro-Gln-Ser; Lys- His-Pro-Gln-Thr; Dha-His-Pro-Gln-Lys; Dhb-His-Pro-Gln-Lys; Lys-His-Pro-Gln-Dha; and Lys-His-Pro-Gln-Dhb. In a preferred embodiment, the sequence is selected from the group consisting of Dha-His-Pro-Gln-Phe-Cys; Dhb-His-Pro-Gln-Phe-Cys; Ser-His-Pro-Gln-Phe-Cys; Thr- His-Pro-Gln-Phe-Cys; Cys-His-Pro-Gln-Phe-Dha; Cys-His-Pro-Gln-Phe-Dhb; Cys-His-Pro-Gln-Phe-Ser; Cys- His-Pro-Gln-Phe-Thr; Dha- His-Pro-Gln-Cys; Dhb-His-Pro-Gln-Cys; Ser-His-Pro-Gln-Cys; Thr-His-Pro-Gln-Cys; Cys- His-Pro-Gln-Dha; Cys-His-Pro-Gln-Dhb; Cys- His-Pro-Gln-Ser and Cys-His-Pro-Gln-Thr.

A further aspect relates to a proteinaceous substance comprising a cyclic affinity tag obtainable by a method of the invention. In one embodiment the proteinaceous substance comprises a lantibiotic-enzyme-mediated cyclized affinity tag. For instance, it comprises a cyclic tag sequence flanked by a dAla-S-Ala, or an Ala-S-dAla or a dAbu-S-Ala or an Ala-S-dAbu or Ala-N-Lys or Lys-N-Ala. In contrast to chemically synthesized thioether-cross linked tags wherein the "bridging" amino acid both have the L stereochemistry, the biologically produced thioether-cyclized tags contain either a D,L- or an LD-thioether-linked ring structure bridging. Therefore, in one embodiment there is provided a proteinaceous substance comprising at least one cyclic tag sequence, the tag sequence being part of a motif containing a D,L- or an LD-thioether-linked ring structure, that bridges (in the N- to C-terminal direction) a D-amino acid and an L-amino acid, or an L-amino acid to a D amino acid, Preferably, the thioether-linked ring structure bridges (in the N- to C-terminal direction) a D-amino acid to an L-amino acid. Also provided is a polynucleotide encoding a proteinaceous substance according to the invention, as well as vectors comprising said polynucleotide. Host cells comprising a polynucleotide or vector according to the invention are also encompassed. These are of particular use for practicing a method of the invention.

As indicated herein above, the cyclic tag may be an external (either N- or C-terminal), internal or intrinsic tag. In case of an external tag, the proteinaceous substance preferably comprises a cleavage site between said polypeptide of interest and the at least one cyclic binding motif. Intrinsic tags are however preferred.

In one embodiment, a proteinaceous substance comprising a cyclic affinity tag obtainable by a method of the invention comprises at least one cyclized streptavidin binding sequence, for instance selected from the group consisting of His-Pro-Gly (HPG), His-Pro-Lys (HPK), His-Pro-Met (HPM), His-Pro-Gln (HPQ), His-Pro-Gln-Phe (HPQF). Lanthionine-enzyme-cyclized streptavidin binding motifs are advantageously used in combination with a (methyl)lanthionine-containing polypeptide of interest. For example, the polypeptide of interest is a member of a library of D,L-thioether-bridged peptides containing thioether-bonds that bridge D- to L-amino acids or L- to D-amino acids, for example a member of a hexa-, hepta- or octapeptide library. In another embodiment, the proteinaceous substance comprises or consists of a non-naturally occurring (mutant) lantibiotic or a lantibiotic fragment comprising a ring structure, and wherein said ring structure comprises at least one cyclic streptavidin binding motif containing a thioether bridge, that bridges (in the orientation N- to C-) a D-amino acid to an L-amino acid, or an L-amino acid to a D amino acid. More than 30 lantibitics have been described in the art (Sahl et al., Annual Reviews in Microbiology, 52, 41-79). They are grouped in two major categories based on their structural features and differences in their modes of action. Type A lantibiotics (e.g. nisin, epidermin and Pep5) are flexible, elongated, amphipathic molecule which mainly act by forming pores in the bacterial cytoplasmic membrane. Type B lantibiotic (e.g. mersacidin) in contrast have a rigid globular shape end inhibit particular enzymes. Lantibiotics are known in the art, and any of those known or yet to be discovered, or a fragment thereof, can be suitably modified such that at least one of the rings contains an affinity tag. Examples include type A lantibiotics, such as nisin or subtilin, epidermin, gallidermin, mutacin 1140, mutacin I, mutacin B-Ny266, ericin A , ericin S, a fragment thereof, such as of nisin's rings ABC, and prenisin(1-45). In one embodiment, the invention provides a mutant lantibiotic wherein ring A is mutated to comprise a cyclic affinity tag sequence, like a HPQ-containing streptavidin-binding sequence. It was surprisingly found that such alteration in the ring structure can be made without abolishing lantibiotic activity. In the nisin mutant G14H, A15P, L16Q, M17F, ΔG18 the five residues of ring C, GALMG, have been replaced by four residues, HPQF. This mutant, HPQF-nisin and also HPQF-nisin Δ(23-34) have growth inhibiting effect of on the indicator strain MG1363. The specific activity is slightly lower than for nisin, but the production level is higher. Accordingly, provided is a mutant lantibiotic wherein at least ring A comprises an affinity tag, preferably wherein the amino acid sequence at positions 4-6 as in native nisin or subtilin is replaced by a streptavidin-binding sequence, like His-Pro-Gln (HPQ). Other potentially active HPQ-lanthionine containing nisin variants have the HPQ sequence in ring A of nisin as in I4H, S5P, L6Q nisin, or having rings DE replaced by one ring with contains the sequence HPQF as in A24H, T25P, C26Q, H27F nisin. Or lacticin A2 variants wherein residues 16-18 (TNT) in ring A are replaced by HPQ. In a further embodiment, at least ring C comprises an affinity tag, preferably wherein the amino acid sequence at positions 14-17 as in native nisin or subtilin is replaced by the sequence His-Pro-Gln-Phe-Gly (HPQFG) or His-Pro-Gln-Phe (HPQF), preferably His-Pro-Gln-Phe (HPQF). In other exemplary mutant lantibiotics, at least rings D and E comprise an affinity tag sequence, preferably wherein the amino acid sequence at positions 24-27 as in native nisin or subtilin is replaced by the sequence His-Pro-Gln-Phe or His-Pro-Gln.

The lantibiotic structure containing the cyclic affinity tag can be used advantageously as external tag for any polypeptide of interest. However, it is particularly suitable to be used in combination with (e.g. fused to) a polypeptide which in itself contains one or more thioether-bridges, for example a therapeutic protein genetically engineered to contain a (methyl)lanthionine to enhance its metabolic stability. By thioether-ring stabilization, the peptides will require less frequent administration and lower doses, while the bioavailability and shelf life may be increased. The attachment of a thioether-ring affinity tag according to the present invention facilitates the purification, detection and/or immobilization of such protein. For example, the cyclized proteinaceous substance is composed of a modified nisin sequence wherein ring C comprises a Streptag sequence, followed by factor X cleavage site and a thioether bridged Ang-(1-7) analog. Exemplary thioether bridged Ang-(1-7) analogs include those disclosed in WO2008/018792. This molecule can be bound to a streptavidine column and released after washing as a purification step with higher yield and higher purity than by conventional hydrophobic interaction. Lantibiotics, or other peptides containing the engineered (methyl)lanthionine-streptavidin binding motif, can be efficiently purified using streptavidin columns, which are commercially available from several companies for instance GE Healthcare, following the protocol of the manufacturer keeping the pH not much higher than 7 (at alkaline pH the dehydroresidues are unstable).

Also provided herein is a library comprising a multiplicity of proteinaceous substances comprising at least one cyclic tag sequence, e.g. a streptavidin binding motif, the tag sequence being part of a motif containing a a D,L- or an LD-thioether-linked ring structure that bridges (in the orientation N- to C-) a D-amino acid and an L-amino acid, or an L-amino acid to a D-amino acid. In addition to the tag sequence, the proteinaceous substance may contain one or more further thioether bridges. For instance, the library comprises a multiplicity of thioether-bridged peptides, each peptide furthermore comprising a lantibiotic-enzyme-introduced thioether-cyclized Strep-tag. Each member of the library may be immobilized on a solid support, preferably in an array format. More preferably, each member is immobilized via the at least one cyclic tag sequence to a suitable (proteinaceous) binding partner spotted in an array format on a solid support. In a specific aspect, the invention provides a library of proteinaceous substances comprising a lantibiotic-enzyme-cyclized Strep-tag sequence, each member being immobilized via the Strep-tag to streptavidin spotted in an array format on a solid support (strep-chip). Also within the scope of the invention is the use of such library for the identification of a peptide sequence of interest, for instance a protein kinase substrate or a receptor ligand.

### LEGENDS TO THE FIGURES

Figure 1. Enzymatic introduction of HPQF in ring C of nisin.
Fig 1A: Mass spectrum of control peptide LMRTTSSLELSDYEQAC before (solid line) and after (dotted line) CDAP modification of a cysteine which yields 25 Da increase in mass.
Fig 1B: *Lactococcus lactis* containing one plasmid encoding H14,P15,Q16,F17,ΔG18 prenisin and a second plasmid encoding NisB, NisT and NisC was induced and the supernatant was analysed by mass spectrometry after addition of TCEP (solid line), which prevents disulfide formation, and CDAP (dotted line), which reacts with free cysteines. Absence of reactivity with CDAP proves lack of availability of the 5 cysteines and closure of all 5 thioether rings.
Figure 2. Antimicrobial activity of a truncated nisin mutant containing HPQF in ringC.
   *Lactococcus lactis* containing a first plasmid encoding NisB, NisT and NisC and a second plasmid encoding MSTKDFNLDLVSVSKKDSGASPRITSISLCTPGCKTHPQFCNMKEQKLISEED was induced. The supernatant was treated with trypsin to cleave off the leader peptide. Series of two-fold dilutions were made in a microwell plate and the peptide's capacity to inhibit the growth of *L. lactis* MG1363 was tested by measuring OD 600 after 6 hours incubation. ○: control with PBS, ■: truncated nisin mutant, ▼: supernatant of nisin A-producing *L. lactis* NZ9700.

### EXPERIMENTAL SECTION

### Example 1 Replacement of the ringC sequence of nisin by HPQF.

### MATERIALS AND METHODS.

Control peptide was LMRTTSSLELSDYEQAC. A nisin mutant was genetically made. In this H14,P15,Q16,F17,AG18 mutant nisin's ring C composed of GALMG was replaced by HPQF. The encoding plasmid was co-expressed with pIl3BTC [Rink, R., et al 2005. Biochemistry 44:8873-8882.]. Mass spec analyses was performed according to [Rink, R., et al 2005. Biochemistry 44:8873-8882]. Disulfide bridge formation was precluded by adding triscarboxyethyl phosphine (TCEP). Treatment with 1-cyano-4-dimethylaminopyridinium tetrafluoroborate (CDAP) was performed to measure the availability of cysteine. If a cysteine is not involved in thioether bridges, it is reactive with CDAP resulting in 25 Da upshift. Absence of 25 Da upshift of a TCEP- and CDAP-treated cysteine-containing peptide indicates the presence of a thioether bridge.

### RESULTS

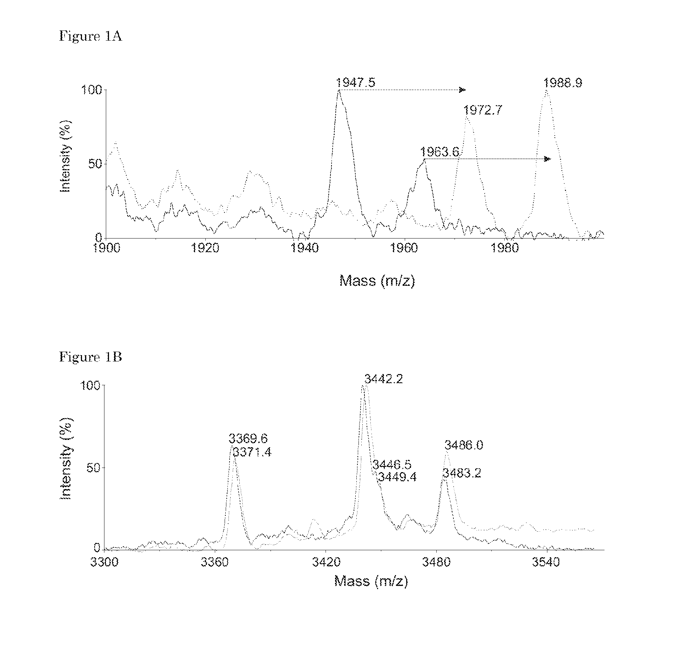

Average mass (m/z) of the unmodified control peptide was 1948.2 Da. Mass spectrometry revealed peaks of 1947.5 Da corresponding to the unmodified peptide and of 1963.6 Da which might correspond to an oxidized form of this peptide; methionine2 might be candidate for the oxidation (Figure 1A, solid line). In the control peptide clearly peaks corresponding to the observed peaks with an increase of 25Da corresponding to CDAP modification (Figure 1A, dotted line). Clearly the procedure proved that the cysteine in the control peptide was available for modification.

The expected mass of fully dehydrated prenisin mutant without methionine-1, containing HPQF was: 5911.8-144 = 5767.8 Da. Peptide with a mass of 5764.2 was observed in the supernatant (not shown). CDAP treatment did not lead to a 25 Da mass increase, as would have been the case for reaction of CDAP with free cysteines. Absence of reactivity indicated that all five cysteines were involved in thioether rings.

To increase the sensitivity of the mass spectrometry by removal of the leader peptide, the supernatant containing the fully dehydrated prenisin mutant comprising motif HPQF was treated with trypsin to cleave off the leader and was analysed (Fig. 1B; solid line prior to CDAP addition, dotted line after CDAP treatment). The expected mass of this nisin mutant after full dehydration and removal of the leader is 3578.2 -144 =3434.2 Da. This value is close to the measured main peak in Fig 1B of 3442 Da. The identity of the two other peaks, 3369 Da and 3486 Da, is not known. Figure 1B demonstrates that addition of CDAP did not cause any shift in mass values. This again proves that all thioether rings were closed. Apparently, the shortened ringC containing HPQF instead of GALMG was very well formed.

### CONCLUSION

Example 1 demonstrates that HPQF can be introduced enzymatically, by the cyclase NisC, in ringC of nisin.

### Example 2. Antimicrobial activity of a truncated nisin variant containing an affinity tag in ring C.

### MATERIALS AND METHODS

A truncated nisin mutant with internal strep tag and external myc tag was genetically constructed. The construct encoded the nisin leader peptide (MSTKDFNLDLVSVSKKDSGASPR) coupled to a H14,P15,Q16,F17,AG 18 mutant of nisin(1-22) i.e. ITSISLCTPGCKTHPQFCNMK, coupled to a myc tag: EQKLISEED. Hence the total presequence was MSTKDFNLDLVSVSKKDSGASPRITSISLCTPGCKTHPQFCNMKEQKLISEED. The peptide was produced by *L. lactis* containing pIl3BTC.

The leader was cleaved off with trypsin and the antimicrobial activity was measured as follows: In a microwell plate: 200 µl of. supernatant of nisin fusion strain and in other wells 200 µl of control (positive e.g. filtered sup of NZ9700 and negative) solutions was added to wells of the first column. 100 µl medium was added to the remaining empty wells. For each row 100 µl from the first column was added to the second and after mixing from the second to the third etc leading to two-fold dilution steps. 100 µl of sensitive strain. MG1363 was added to all wells at low density, OD600: 0.05-0.1. After 6 hours OD600 was measured.

### RESULTS

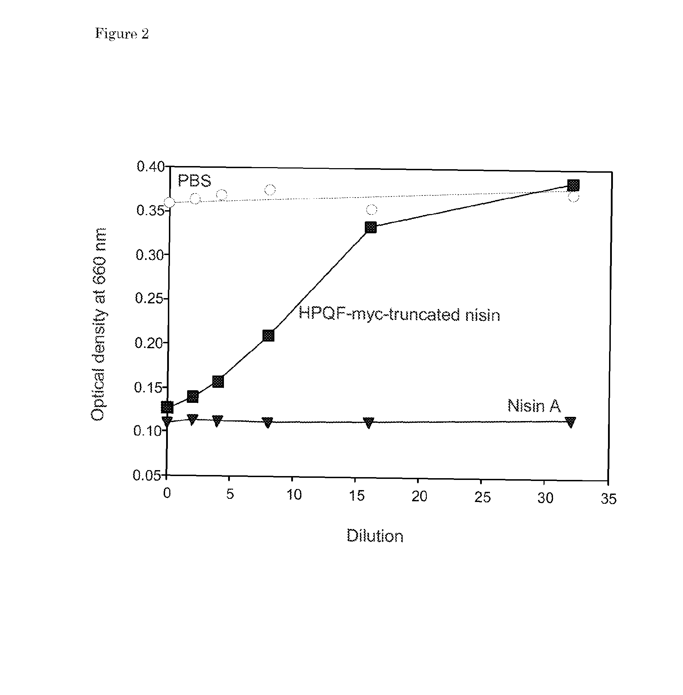

Figure 2 shows that the truncated nisin's variant with HPQF-containing ringC has clearly antimicrobial activity.

### CONCLUSION

Example 2 demonstrates that the presence of HPQF in ring C does not abolish the antimicrobial activity of the truncated nisin mutant. This is surprising because simultaneously four amino acids are mutated whereas one amino acid is deleted. Despite the truncation (which may already lead to 10-fold reduction), despite the shortened ring C and despite the negatively charged tail (which reduces binding to the anionic membrane), the peptide still has activity.

## Claims

1. An enzymatic method for providing a proteinaceous substance comprising a polypeptide of interest and a cyclic affinity tag, comprising the steps of:
a) providing at least one precursor proteinaceous substance, the precursor comprising said protein of interest and at least one motif of the general formula X1-Tag-X2 wherein
X1 and X2 represent amino acids whose side chains can be linked enzymatically by a covalent bond;
Tag is an amino acid sequence capable of binding to a binding partner when cyclized;
b) contacting said precursor with at least one enzyme capable of forming a covalent bond between X1 and X2, thereby introducing an intramolecular ring structure comprising the Tag sequence; and
c) isolating the resulting cyclized proteinaceous substance.

2. Method according to claim 1, wherein said polypeptide of interest is fused N- or C-terminally to the at least one at least one motif of the general formula X1-Tag-X2.

3. Method according to 2, wherein the proteinaceous substance comprises a cleavage site between said polypeptide of interest and the at least one motif, preferably wherein the cleavage site is a Factor X or a Glu-C -cleavage site.

4. Method according to claim 3, wherein step c) is followed by cleavage of the cyclized proteinaceous substance at the cleavage site to releasing the polypeptide of interest

5. Method according to claim 1, wherein said proteinaceous substance is a polypeptide of interest wherein a portion is replaced by said at least one motif such that the motif is an integral part of said polypeptide of interest.

6. Method according to any one of the preceding claims, wherein steps a) and b) are performed in a host cell comprising said at least one enzyme capable of forming a covalent bond between X1 and X2, said host cell being provided with a nucleic acid sequence encoding said precursor proteinaceous substance.

7. Method according to any one of the preceding claims wherein Tag comprises a streptavidin binding sequence or the sequence RGD, preferably wherein the streptavidin binding sequence is selected from the group consisting of His-Pro-Gly (HPG), His-Pro-Lys (HPK), His-Pro-Met (HPM), His-Pro-Gln (HPQ) and His-Pro-Gln-Phe (HPQF), preferably wherein the streptavidin binding sequence is His-Pro-Gln (HPQ) or His-Pro-Gln-Phe (HPQF).

8. Method according to any one of the preceding claims, wherein step a) comprises providing a precursor proteinaceous substance wherein X1 is selected from Dhb, Dha, Thr, and Ser and wherein X2 is Cys or Lys; or wherein X1 is Cys or Lys and X2 is selected from Dhb, Dha, Thr and Ser, and wherein said motif is preceded N-terminally by a lantibiotic leader sequence; and wherein the precursor is contacted in step b) is a lantibiotic enzyme capable of forming a thioether bridge is formed between residues X1 and X2.

9. Method according to claim 8, wherein the motif X1-Tag-X2 consists of an amino acid sequence selected from the group consisting of Dha- His-Pro-Gln-Phe-Cys; Dhb-His-Pro-Gln-Phe-Cys; Ser- His-Pro-Gln-Phe-Cys; Thr- His-Pro-Gln-Phe-Cys; Cys- His-Pro-Gln-Phe-Dha; Cys- His-Pro-Gln-Phe-Dhb; Cys- His-Pro-Gln-Phe-Ser; Cys- His-Pro-Gln-Phe-Thr; Dha- His-Pro-Gln-Cys; Dhb- His-Pro-Gln-Cys; Ser- His-Pro-Gln-Cys; Thr- His-Pro-Gln-Cys; Cys- His-Pro-Gln-Dha; Cys- His-Pro-Gln-Dhb; Cys- His-Pro-Gln-Ser; Cys- His-Pro-Gln-Thr; Ser- His-Pro-Gln-Phe -Lys; Thr- His-Pro-Gln-Phe-Lys; Lys- His-Pro-Gln-Phe -Ser; Lys-His-Pro-Gln-Phe-Thr; Dha- His-Pro-Gln-Phe - Lys; Dhb- His-Pro-Gln-Phe-Lys; Lys- His-Pro-Gln -Dha; Lys- His-Pro-Gln-Dhb; Ser-His-Pro-Gln -Lys; Thr- His-Pro-Gln Lys; Lys- His-Pro-Gln-Ser; Lys- His-Pro-Gln-Thr; Dha- His-Pro-Gln -Lys; Dhb-His-Pro-Gln-Lys; Lys-His-Pro-Gln-Dha; and Lys- His-Pro-Gln-Dhb.

10. Method according to claim 8 or 9, wherein step b) comprising contacting the precursor with lantibiotic enzyme LanM, cyclase LanC (in the case of a combination of a dehydroresidue and a cysteine) or a combination of a lantibiotic dehydratase LanB and cyclase LanC, preferably wherein steps a) and b) are performed in a host cell comprising lanthionine proteins LanB; LanC and LanT; LanM and LanT; LanB and LanC; or LanM

11. Method according to claim 1-10, wherein the polypeptide of interest naturally comprises at least one thioether-containing intramolecular ring structure.

12. Proteinaceous substance comprising a cyclic tag sequence, obtainable by a method according to any one of claims 1-11.

13. A proteinaceous substance comprising at least one cyclic tag sequence, the tag sequence being part of a thioether-linked ring structure bridging (in the orientation N- to C-) a D-amino acid and an L-amino acid, or an L-amino acid to a D amino acid.

14. Proteinaceous substance according to claim 13, wherein said cyclic tag sequence is cyclized streptavidin binding motif, preferably comprising or consisting of a sequence selected from the group consisting of His-Pro-Gly (HPG), His-Pro-Lys (HPK), His-Pro-Met (HPM), His-Pro-Gln (HPQ) and His-Pro-Gln-Phe (HPQF).

15. Proteinaceous substance according to claim 13 or 14, comprising a mutant lantibiotic or a lantibiotic fragment comprising a ring structure, and wherein said ring structure comprises at least one cyclic streptavidin binding motif containing a thioether bridge, that bridges (in the orientation N- to C-) a D-amino acid to an L-amino acid, or an L-amino acid to a D- amino acid.

16. A peptide library comprising a multiplicity of proteinaceous substances according to any one of claims 12-15.

17. Library according to claim 16, wherein each member is immobilized on a solid support, preferably in an array format, more preferably wherein each member is immobilized via the at least one cyclic streptavidin binding motif to streptavidin spotted in an array format on a solid support.
